Europäisches Patentamt

(19) European Patent Office

Office européen des brevets

(11) Publication number : **0 492 920 A1**

# EUROPEAN PATENT APPLICATION

(21) Application number : 91311660.4

(22) Date of filing : 16.12.91

(51) Int. Cl.⁵ : **C12N 15/44, A61K 39/145, A61K 39/21, C07K 15/00, C12N 15/62**

(30) Priority : 24.12.90 US 632683

(43) Date of publication of application :
01.07.92 Bulletin 92/27

(84) Designated Contracting States :
CH DE FR GB IT LI NL

(71) Applicant : MERCK & CO. INC.
126, East Lincoln Avenue P.O. Box 2000
Rahway New Jersey 07065-0900 (US)

(72) Inventor : Tolman, Richard L.
29 Upper Warren Way
Warren, NJ 07059 (US)
Inventor : Underwood, Dennis J.
247 East 4th Avenue
Roselle, NJ 07203 (US)

(74) Representative : Thompson, John Dr. et al
Merck & Co., Inc. European Patent
Department Terlings Park Eastwick Road
Harlow, Essex CM20 2QR (GB)

(54) Chimaeric influenza-HIV vaccine.

(57) The exposed and highly immunogenic A-antigenic loop of influenza hemagglutinin (HA) is homologous with a Principal Neutralizing Determinant (PND) present on the Human Immunodeficiency Virus' (HIV) envelope glycoprotein, gp120. Inoculation of a mammal with a killed or attenuated virus, or purified HA protein, modified so as to convert the HA A-antigenic site into an HIV PND, produces the usual strong response to the HA site A, but the antibody produced is specific to the hemagglutinin-HIV PND chimaera, and is useful to neutralize HIV.

EP 0 492 920 A1

## BACKGROUND OF THE INVENTION

Acquired Immune-Deficiency syndrome (AIDS), AIDS Related Complex (ARC), and other related pathologies associated with Human Immunodeficiency Virus (HIV) infections and disease are reaching epidemic proportions. Efforts to limit continued spread of the virus include production and testing of antiretroviral compounds on the one hand, and prophylactic compounds capable of inducing protective immune responses, on the other.

Efforts at producing effective anti-HIV vaccines include preparations comprising whole killed HIV, HIV protein subunits, HIV Principal Neutralizing Determinant (PND) peptides and conjugates thereof, and recombinant vaccinia virus vectors expressing HIV antigens. The following references provide a general overview of ongoing vaccine evaluations: Lasky, L. A., Crit. Rev. in Immunol. 9, 153 (1989); Garrison, L., Clements, L. M., Comprehensive Therapy 15, 47 (1989); Dalgleish, A., Drugs of Today, 25, 709 (1989); Schulhafer, E. P., Verma, R. S., In Vivo 3, 61 (1989); Fauci, A. S., et al., Annals of Internal Medicine 110, 373 (1989); Rosenberg, Z. F., Fauci, A. S., Advances in Immunol. 47, 377 (1989); and Snart, R. S. AIDS 2, S107 (1988). Evans, et al., [Nature, 339, 385 (1989)] and more recently Crabbe et al., [FEBS. Lett., 271, 194 (1990)] have prepared poliovirus chimaeras comprising HIV epitopes.

This invention provides a novel solution to the anti-HIV vaccine problem by disclosing an influenza hemagglutinin (HA) molecule modified so as to form an influenza-HIV PND chimaera. This novel molecule is then susceptible to presentation to a human at risk of either HIV or influenza infection as a component of an attenuated or killed influenza virus, or as an isolated protein immunogen as a component of a pharmaceutically acceptable composition. Whereas poliomyelitis has been almost completely eradicated, influenza is still a prevalent disease, especially in the elderly and in immunocompromised individuals. Thus, this invention provides a product capable of preventing, ameliorating, or treating two very common diseases, one of which is lethal.

Influenza viruses of subtypes A, B, and C are members of the orthomyxoviridae family. These viruses have a negative-strand RNA genome comprising 8 separate and unique RNA segments, coding for 10 proteins, including neuraminidase and hemagglutinin. It is well established that host antibodies are directed against hemagglutinin, the cell surface receptor binding protein [Porter, et al., Nature 282, 471 (1979); Krug, R. M., The Influenza viruses, Plenum Press, 1989].

Mutant strains of the A subtype have been classified on the basis of the type of hemagglutinin and/or neuraminidase found in the isolate. Thus, the nomenclature for human influenza A virus, based on the identity of the surface antigens, includes variants of the H1N1 (1918-1957), H2N2 (1957-1968), and H3N2 (1968-) subtypes [Fang, R. et al., Cell 25, 315 (1981)].

This invention makes use of the known antigenic character of the hemagglutinin molecule, the availability of known technology for production of attenuated or killed influenza virus, and the presence on the hemagglutinin molecule of the prominent A antigenic site which in some influenza isolates is remarkably homologous to the HIV PND [Wiley, D.C. et al., Nature 289, 373 (1981)], to generate a chimaeric influenza-HIV PND vaccine.

## SUMMARY OF THE INVENTION:

A human influenza virus strain, such as an H3N2 isolate, is mutagenized so as to convert the hemagglutinin A antigenic site into an HIV PND. The live attenuated virus, the whole killed virus, or the chimaeric hemagglutinin-HIV PND protein is then used as an immunogen to elicit anti-HIV PND, anti-hemagglutinin, anti-influenza, or HIV-neutralizing immune responses in mammalian recipients.

## DETAILED DESCRIPTION

The novel HIV PND bearing live-attenuated or killed influenza virus of this invention is prepared by any of a number of processes. A general knowledge of molecular biological techniques known in the art is assumed, and the reader is referred to the following excellent texts for detailed descriptions of techniques used herein: Sambrook, J., et al., Molecular Cloning: A Laboratory Manual, Cold spring Harbor Laboratory Press, New York (1989); Ausubel, F. M., et al., Current Protocols in Molecular Biology, Wiley and Sons, New York (1987). In one process, an attenuated influenza virus is cultured in vitro in the presence of a mutagen. Mutants having the desired HIV PND peptide sequence are identified by immunological screening with an anti-HIV PND antibody or by in-situ hybridization of the mutant viral RNA's with a nucleic acid probe complementary to the desired HIV PND sequence.

Alternatively, the viral hemagglutinin RNA of an influenza virus is isolated, reverse transcribed to generate a cDNA copy, and inserted into a DNA vector [Evans, D. J., et al., Nature 339, 385 (1989); Jou, W. M., et al., Cell 19, 683 (1980); Emtage, J. S. and Carey, N. H., US Patent 4,357,421; Daniels, R. S., et al., J. Gen. Virol.

66, 457 (1985); Porter, A. G., et al., Nature 282, 471 (1979); Gething, M., et al., Nature 287, 301 (1980); Winter, G., et al., Nature 292, 72 (1981); Laver, W. G., et al., Nature 283, 454 (1980)]. A portion of the hemagglutinin gene is subcloned into a vector suitable for site-directed mutagenesis, and clones bearing the desired sequence are selected for as described above. The hemagglutinin-HIV PND coding DNA is subcloned into a vector carrying RNA polymerase control signals and an RNA copy produced. The mutant RNA is isolated, reconstituted [Yamanka, K., et al., J. Biol,. Chem. 265, 11151 (1990)] with the other eight viral RNA's and viral proteins, and introduced into a host cell by any of the known methods of electroporation, microinjection, or encapsidation and infection. The mutant virus shed from the host cells in culture is harvested and killed or attenuated according to known procedures [Gabliks, Janis, US Patent 4,783,411; Wright, P. F., and Karzon, D. T., Prog. Med. Virol. 34, 70 (1987)] and used as an anti-HIV vaccine.

In yet another alternate method, the hemagglutinin-HIV PND coding DNA produced as described above is subcloned into an expression vector and chimaeric hemagglutinin-HIV PND gene product is isolated. This isolated protein is included in a pharmaceutically acceptable composition to form an anti-HIV/anti-influenza subunit vaccine.

Whatever the mutagenesis method selected, choice of an influenza virus having an hemagglutinin with an A site homologous to the HIV PND minimizes the requisite number of nucleotide changes. Table I below shows a prevalent HIV PND peptide sequence and the possible base sequences that could code for that sequence. It should be understood that other possible related HIV PND sequences are produced by making nucleotide changes as for the exemplary sequence shown below. Thus, HIV PNDs comprising any of the sequences -GlyProGlyArg- (SEQ ID: 1:), -GlyProGlyArgAla- (SEQ ID: 2:), -GlyProGlyArgAlaPhe- (SEQ ID: 3:), -GlyProGlyArgVallIle- (SEQ ID: 4:), -GlyProGlyArgAlaIle- (SEQ ID: 5:), -GlnArgGlyProGlyArgAlaPhe- (SEQ ID: 6:), -HisIleGlyProGlyArgAlaPhe- (SEQ ID: 7:) are all amenable to being incorporated into the influenza hemagglutinin by the methods provided herein. Below the sequence of the HIV PND, the amino acid sequence of the hemagglutinin A-antigenic site from a number of isolates is shown. The minimum number of base changes required for the A-site to have the primary sequence of the sample HIV PND is also shown.

## Table I

| Strain | Peptide Sequence Base Sequence[a] | Ref. | # changes to form HIV PND | SEQ ID |
|---|---|---|---|---|
| HIV PND | -GlyProGlyArg- | I. | | 1 |
| | -GGNCCNGGNCGN- | | | 12 |
| | -GGNCCNGGNAGR- | | 0 | 13 |
| A/Duck/Ukraine/63 | -GlyProAlaAsn- | | | 8 |
| | -GGACCTGCTAAC- | II. | | 14 |
| CHANGE TO: | -GGACCTGGTAGR- | | 3 | 15 |
| A/Aichi/2/68 | -GlyProGlySer- | | | 9 |
| | -GGACCTGGTAGC- | II. | | 16 |
| CHANGE TO: | -GGACCTGGTAGR- | | 1 | 17 |
| A/Victoria/3/75 | -GlyProAspSer- | | | 10 |
| | -GGACCTGATAGC- | III. | | 18 |
| CHANGE TO: | -GGACCTGGTAGR- | | 2 | 19 |

3

---

References and Footnotes:

I.     Javaherian, K., et al., PNAS USA 86, 6768 (1989).

II.    Fang, Cell 25, 315, 1981

III.   Verhoyen, Nature 286, 771, 1980.

a    Ambiguity Code for bases:

R = A or G

N = G, A, T, or C

Y = C or T

---

Identical to the Aichi/2/68 strain is the Mem/1/71 strain, while the Eng/878/69, Eng/187/70, Eng/42/72, Mem/102/72, and PC/1/73 strains are identical to the A/victoria/3/75 strain in this region of the molecule. Thus, as little as one nucleotide change is required to convert the primary sequence of the hemagglutinin A antigenic site into a prevalent HIV PND. Naturally, as the amino acid sequence extends, on either side, from the core sequence -GlyProGlyArg- (SEQ ID: 1), more changes are required to maintain homology with specific HIV strains.

It is also noteworthy that the required changes between the influenza A-site and the HIV PND are largely conservative. Where the change is from Ala to Gly, a neutral, sterically unhindered amino acid replaces another neutral, unhindered amino acid. The replacement of Ser or Asn with Arg is a somewhat less conserved change, representing the addition of a positive charge to this portion of the hemagglutinin, but the sizes of the amino acid side groups are once again roughly similar. In short, the changes required are not likely to produce a substantial alteration in the overall conformation of hemagglutinin, especially given the already exposed and isolated location of the A-antigenic site.

Good evidence exists that the presence of the tetrapeptide -GlyProGlyArg- (SEQ ID: 1), found in a great many HIV strains, is sufficient to induce HIV neutralizing immune responses in mammals [Goudsmit, J., AIDS 2(suppl. 1):S41-S45 (1988); Goudsmit, J., et al., Res. Virol. 140, 419 (1989); Javaherian, K., et al., PNAS USA 86, 6768 (1989); LaRosa, G. J., et al., Science 249, 932 (1990)]. Furthermore, strong evidence exists that short peptides from the hemagglutinin A-antigenic site are capable of eliciting strong antibody responses [Muller, G. M., et al., PNAS USA 79, 569 (1982); Schulze-Gahmen, U., et al., Eur. J. Biochem. 159, 283 (1986); Muller, S., et al., Vaccine 8, 308 (1990)]. Thus, this region should maintain its antigenicity, especially given the conservative nature of the required changes.

Production of a chimaeric virus, live attenuated or killed is achieved by mutagenesis of the whole virus and isolation of strains coding for the desired HIV PND amino acid sequence. Such an approach requires at least two separate genetic mutations to occur in the same molecule in a desirable region of the RNA genome. Although not theoretically impossible to achieve, such an approach is labor intensive and subject to chance. More preferably, the desired genetic alterations are introduced by a site-directed mutagenesis route. Accordingly, a complimentary DNA copy (cDNA) of the hemagglutinin viral RNA, is synthesized according to methods known in the art [US Patent 4,357,421; Sambrook, J., et al., Molecular Cloning: A Laboratory Manual, Cold Spring Harbor Laboratory Press, New York (1989); Ausubel, F. M., et al., Current Protocols in Molecular Biology, Wiley and Sons, New York (1987)]. Mutagenesis of the cDNA and reconstitution [Yamanaka, K., et al., J. Biol. Chem. 265, 11151 (1990)] of a chimaeric viral transcript with other viral proteins and RNAs of the influenza genome allows propagation of an infectious particle for attenuated live or killed virus vaccine production.

Alternatively, a chimaeric hemagglutinin-HIV PND protein may be produced by expression of the mutagenized hemagglutinin DNA in a bacterial, yeast, or mammalian expression system. Davis et al., [Gene 21, 273 (1983)] showed that wild-type hemagglutinin protein produced in E. coli was capable of inducing mammalian antibodies which bound the HA protein recombinant, detergent-treated viral HA, HA on intact virions, and HA on the surface of cells infected with influenza virus. There is every reason to suppose that the hemagglutinin-HIV PND recombinant chimaera will be as effective in inducing anti-hemagglutinin and anti-HIV PND antibodies.

Once a chimaeric virus has been prepared, it may be killed or attenuated according to methods known in the art [La Montagne, J. R. et al., Reviews of Infect. Dis. 5, 723 (1983): Clements, M. L. et al.,Lancet, 705 (March 31, 1984): Quinnan, G. V. et al., Rev. Infect. Dis. 5, 748 (1983); Murphy, B. R. et al., Infection and Immunity 73, 235 (1982); Tolpin M. D., et al., Infection and Immunity 36, 645 (1982); Gabliks, J. US Patent 4,783,411]. Alternatively, a chimaeric hemagglutinin-HIV PND protein may be utilized. Such a viral antigen may be formulated into a composition comprising an inert carrier, adjuvant compounds, or immunomodulatory compounds such as those, for example, reported in Market Letter, Nov. 30, 1987, pp. 26-27, and Genetic Engineering News, Jan. 1988, Vol. 8, p23. The composition and should contain from 1 microgram to 1 milligram of attenuated or killed virus or chimaeric hemagglutinin protein per milligram of composition. An immunologically effective dose based on total protein is administered, and should be in the range of between 0.1 microgram and 10 milligrams of protein per kilogram of recipient body weight. The dose may be delivered intranasally, subcutaneously, intradermally, intravenously, or intramuscularly. A booster dose should be administered whenever anti-hemagglutinin-HIV PND chimaera antibodies decline, and preferably on a yearly to five yearly basis.

The following examples are provided by way of illustration only and should not be construed as limiting the invention.

## EXAMPLE 1

### Mutagenesis of Influenza Hemagglutinin Encoding DNA to Produce a Chimaeric Hemagglutinin-HIV PND Gene:

A cDNA copy of influenza virus RNA is prepared essentially according to the method disclosed in US Patent 4,357,421, and the following description. High yielder recombinant strain X31, carrying the hemagglutinin and neuraminidase genes of influenza virus strain Aichi/2/68, is cultured in embryonated chicken eggs, the virus concentrated by ultracentrifugation through sucrose, and the viral RNA extracted according to the process of Palese, P. and Schulman, J. L., J. Virol. 17, 876 (1976). The RNA is polyadenylated using $^{14}$C-ATP, and converted to double stranded DNA, according to the method of Devos et al., Eur. J. Biochem. 62, 401 (1976); J. Mol. Biol. 128, 595 (1979). Gel isolated dsDNA is isolated ard tailed by polynucleotide terminal transferase and TTP, and cloned into the Pst1 site of plasmid pGEM®5Zf(+ or -) [Promega Biotech, catalog #P2241 or P2351] by the poly(dA).poly(dT) tailing method. White E. coli colonies are selected after transforming E. coli JM109 with the plasmid DNA and plating on X-gal and IPTG indicator and ampicillin containing agar plates. The plasmid DNA is isolated by miniprep, digested with Pst1, and the fragments run on a 2% agarose gel. The presence of hemagglutinin gene containing clones is confirmed by southern hybridization of the gel and probing with the $^{32}$P-polynucleotide kinase labelled single-stranded oligonucleotide -GGACCTGGTAGCGGTTTT- (SEQ ID: 20), which codes for the hemagglutinin A-site epitope -GlyProGlyserGlyPhe- (SEQ ID: 11).

Hemagglutinin bearing clones are grown up and superinfected with helper bacteriophage. The encapsidated single stranded DNA is recovered from the culture medium and purified by phenol extraction followed by caesium-chloride banding. The sequence of the A-antigenic site is mutagenized by hybridization of the single stranded DNA with the oligonucleotide -GGACCTGGTAGAGCTTTT- (SEQ ID: 21), which codes for the HIV PND and introduces an AluI restriction site (AG CT) into the DNA at this location. The complete double stranded circle is synthesized by addition of DNA polymerase in the presence of all four deoxynucleotide triphosphates, ATP, and DNA ligase. The reaction product is transformed into JM109, white colonies are selected, and miniprep DNA prepared.

Clones carrying mutagenized hemagglutinin-HIV PND chimaeric DNA are detected by digestion of miniprep DNA with HindIII, which linearizes the vector [there is no HindIII site in the pGEM5Zf(+ or -) vector and there is only one HindIII site in the hemagglutinin gene, located 149 bases to the 5'-side of the mutagenized site]. The recessed 3'-ends of the linearized DNA, are labelled by addition of the Klenow fragment of DNA polymerase in the presence of $^{32}$P-dATP, followed by digestion with AluI. Electrophoresis of the reaction products of a wild-type molecule on a 5% acrylamide gel followed by autoradiography reveals a labelled fragment of 675 base-pairs, and of 519 base-pairs. On the other hand, a mutagenized clone has a new AluI site introduced into the 519 base-pair fragment, thus yielding labelled fragments of 675 base-pairs and 149 base-pairs. Clones revealing the latter pattern code for the chimaeric influenza hemagglutinin-HIV PND product.

## EXAMPLES 2

### Preparation of Chimaeric Influenza Hemagglutinin-HIV PND RNA:

The chimaeric DNA construct prepared in Example 1 is linearized on the distal end of the insert with respect to one of the vector's RNA polymerase promoter signals. For this purpose, any of the restriction enzymes that do not cut inside the insert but which are present in the vector's multiple cloning site may be used. Coding RNA is obtained by using a pGEM®-5Zf(+ or -) clone from Example 1 oriented such that the chimaeric Pst1 insert's noncoding strand 5′-end is closest to the T7-promoter. The vector is linearized with AatII, NcoI, SacII, EcoRV, or NotI, and transcription is initiated by addition of SP6 RNA polymerase. Where the Pst1 insert is oppositely orineted, coding RNA is obtained by digestion with salI to allow runoff transcription to be commenced from the T7 promoter site.

EXAMPLE 3

Preparation of an Influenza Virus-HIV PND Chimaera through Reconstitution of Chimaeric Hemagglutinin-HIV PND RNA with Wild-Type Influenza Virus Constituents:

The RNA transcript obtained from Example 2 is purified by phenol extraction and ethanol precipitation. RNA from wild type influenza is isolated as described in Example 1, and the wild-type hemagglutinin RNA is removed, either by electrophoresis of the wild type RNA and electroelution of all but the hemagglutinin coding RNA, or by hybridization depletion using immobilized complimentary single stranded hemagglutinin DNA. The hemagglutinin depleted RNA is quantitated and then mixed with an approximately 1:1 molar amount of quantitated chimaeric RNA transcript from Example 2. The RNA mixture is reconstituted with influenza virus proteins to form virions. Virus is cultured in embryonated chicken eggs and either killed or attenuated for use as an anti-influenza, anti-HIV chimaeric vaccine.

EXAMPLE 4

Chimaeric Recombinant Influenza Hemagglutinin-HIV PND Protein:

The isolated plasmid DNA prepared according to Example 1 is digested with Pst1, and the approximately 2000 base-pair fragment is isolated by electrophoresis through a 1% agarose gel and electroelution of the approximately 2000 base pair fragment. The Pst1 fragment is trimmed back by treatment with exonuclease (Exonuclease III or BAL 31) to remove excess nucleotides. The blunt ended fragments are then subcloned into a bacterial expression vector under control of the trp operon, transformed into E. coli, and expression of the chimaeric hemagglutinin-HIV PND protein initiated by tryptophan starvation. The protein is purified according to the process disclosed by Davis, A. R., et al., Gene 21, 273 (1983)].

SEQUENCE LISTING

(2)  INFORMATION FOR SEQ ID NO:  1 :
     (i)      SEQUENCE CHARACTERISTICS:
              (A)  LENGTH:  4 amino acids
              (B)  TYPE:  amino acid
              (C)  STRANDEDNESS:  _____
              (D)  TOPOLOGY:  unknown
     (ii)     MOLECULE TYPE:  protein
     (v)      FRAGMENT TYPE:  internal
     (vi)     ORIGINAL SOURCE:  _____
              (A)  ORGANISM:  Human Immunodeficiency Virus
              (B)  STRAIN:  several
     (vii)    IMMEDIATE SOURCE:  Mutagenesis
     (viii)   POSITION IN GENOME:  Within ENV
     (ix)     FEATURE:  _____
              (A)  NAME/KEY:  SEQ ID NO 1 is part of one
                             principal-neutralizing
                             determinant of HIV
              (B)  LOCATION:  Within ENV
     (xi)     SEQUENCE DESCRIPTION:  SEQ ID NO:  1 :
              Gly Pro Gly Arg
              1


(2)  INFORMATION FOR SEQ ID NO:  2 :
     (i)      SEQUENCE CHARACTERISTICS:
              (A)  LENGTH:  5 amino acids
              (B)  TYPE:  amino acid
              (C)  STRANDEDNESS:  _____
              (D)  TOPOLOGY:  unknown
     (ii)     MOLECULE TYPE:  protein
     (v)      FRAGMENT TYPE:  internal
     (vi)     ORIGINAL SOURCE:  _____
              (A)  ORGANISM:  Human Immunodeficiency Virus
              (B)  STRAIN:  several
     (vii)    IMMEDIATE SOURCE:  Mutagenesis

(viii)  POSITION IN GENOME: Within ENV

(ix)    FEATURE: _____

    (A)  NAME/KEY:  SEQ ID NO 2 is part of one

                        principal-neutralizing

                        determinant of HIV

    (B)  LOCATION:  Within ENV

(xi)    SEQUENCE DESCRIPTION:  SEQ ID NO:  2 :

Gly Pro Gly Arg Ala

1             5


(2)  INFORMATION FOR SEQ ID NO:  3 :

(i)     SEQUENCE CHARACTERISTICS:

    (A)  LENGTH:  6 amino acids

    (B)  TYPE:  amino acid

    (C)  STRANDEDNESS: _____

    (D)  TOPOLOGY:  unknown

(ii)    MOLECULE TYPE: protein

(v)     FRAGMENT TYPE:  internal

(vi)    ORIGINAL SOURCE: _____

    (A)  ORGANISM:  Human Immunodeficiency Virus

    (B)  STRAIN:  several

(vii)   IMMEDIATE SOURCE: Mutagenesis

(viii)  POSITION IN GENOME: Within ENV

(ix)    FEATURE: _____

    (A)  NAME/KEY:  SEQ ID NO 3 is part of one

                        principal-neutralizing

                        determinatn of HIV

    (B)  LOCATION:  Within ENV

(xi)    SEQUENCE DESCRIPTION:  SEQ ID NO:  3 :

Gly Pro Gly Arg Ala Phe

1             5

(2)  INFORMATION FOR SEQ ID NO:  4 :

    (i)       SEQUENCE CHARACTERISTICS:

           (A)   LENGTH:  6 amino acids

           (B)   TYPE:  amino acid

           (C)   STRANDEDNESS:  _____

           (D)   TOPOLOGY:  unknown

    (ii)      MOLECULE TYPE:  protein

    (v)       FRAGMENT TYPE:  internal

    (vi)      ORIGINAL SOURCE:  _____

           (A)   ORGANISM:  Human Immunodeficiency Virus

           (B)   STRAIN:  several

    (vii)     IMMEDIATE SOURCE:  Mutagenesis

    (viii)    POSITION IN GENOME:  Within ENV

    (ix)      FEATURE:  _____

           (A)   NAME/KEY:  SEQ ID NO 4 is part of one

                        principal—neutralizing

                        determinant of HIV

           (B)   LOCATION:  Within ENV

    (xi)      SEQUENCE DESCRIPTION:  SEQ ID NO:  4 :

           Gly Pro Gly Arg Val Ile

           1                5


(2)  INFORMATION FOR SEQ ID NO:  5 :

    (i)       SEQUENCE CHARACTERISTICS:

           (A)   LENGTH:  6 amino acids

            (B)   TYPE:  amino acid

           (C)   STRANDEDNESS:  _____

            (D)   TOPOLOGY:  unknown

    (ii)      MOLECULE TYPE:  protein

    (v)       FRAGMENT TYPE:  internal

    (vi)      ORIGINAL SOURCE:  _____

           (A)   ORGANISM:  Human Immunodeficiency Virus

           (B)   STRAIN:  several

    (vii)     IMMEDIATE SOURCE:  Mutagenesis

```
        (viii)  POSITION IN GENOME: Within ENV
        (ix)    FEATURE:    _____
                (A)  NAME/KEY:   SEQ ID NO 5 is part of one
                                 principal-neutralizing___
                                 determinant of HIV_____
                (B)  LOCATION:  Within ENV_____
        (xi)    SEQUENCE DESCRIPTION:  SEQ ID NO:  _5_:
                Gly Pro Gly Arg Ala Ile
                1              5


(2)  INFORMATION FOR SEQ ID NO:  _6_:
        (i)     SEQUENCE CHARACTERISTICS:
                (A)  LENGTH:  8 amino acids_____
                (B)  TYPE:  amino acid_____
                (C)  STRANDEDNESS:  _____
                (D)  TOPOLOGY:  unknown_____
        (ii)    MOLECULE TYPE: protein_____
        (v)     FRAGMENT TYPE:  internal_____
        (vi)    ORIGINAL SOURCE:  _____
                (A)  ORGANISM:  Human Immunodeficiency Virus
                (B)  STRAIN:  several_____
        (vii)   IMMEDIATE SOURCE: Mutagenesis_____
        (viii)  POSITION IN GENOME: Within ENV
        (ix)    FEATURE:    _____
                (A)  NAME/KEY:   SEQ ID NO 6 is part of one
                                 principal-neutralizing___
                                 determinant of HIV_____
                (B)  LOCATION:  Within ENV_____
        (xi)    SEQUENCE DESCRIPTION:  SEQ ID NO:  _6_:
                Gln Arg Gly Pro Gly Arg Ala Phe
                1              5
```

(2)  INFORMATION FOR SEQ ID NO: __7_ :

    (i)        SEQUENCE CHARACTERISTICS:

            (A)  LENGTH:  8 amino acids _____

            (B)  TYPE:  amino acid _____

            (C)  STRANDEDNESS: _____

            (D)  TOPOLOGY:  unknown _____

    (ii)       MOLECULE TYPE: protein _____

    (v)        FRAGMENT TYPE:  internal _____

    (vi)       ORIGINAL SORUCE: _____

            (A)  ORGANISM:  Human Immunodeficiency Virus_

            (B)  STRAIN:  several _____

    (vii)      IMMEDIATE SOURCE: Mutagenesis _____

    (viii)  POSITION IN GENOME: Within ENV

    (ix)       FEATURE: _____

            (A)  NAME/KEY:  SEQ ID NO 7 is part of one

                            principal-neutralizing ___

                            determinant of HIV _____

            (B)  LOCATION:  Within ENV _____

    (xi)       SEQUENCE DESCRIPTION:  SEQ ID NO: __7_ :

            His Ile Gly Pro Gly Arg Ala Phe

            1                5


(2)  INFORMATION FOR SEQ ID NO: __8_ :

    (i)        SEQUENCE CHARACTERISTICS:

            (A)  LENGTH:  5 amino acids _____

            (B)  TYPE:  amino acid _____

            (C)  STRANDEDNESS: _____

            (D)  TOPOLOGY:  unknown _____

    (ii)       MOLECULE TYPE: protein _____

    (v)        FRAGMENT TYPE:  internal _____

    (vi)       ORIGINAL SOURCE: _____

            (A)  ORGANISM:  Influenza Virus_

            (B)  STRAIN:  several _____

(vii)   IMMEDIATE SOURCE: A/Duck/Ukraine/63 influenza virus

(viii)  POSITION IN GENOME: A-antigenic site of hemagglutinin molecule

(ix)    FEATURE: _____

    (A)  NAME/KEY:  SEQ ID NO 8 is part of the

                    influenza virus A-antigenic site

                    which is homologous to an HIV

                    principal-neutralizing determinant

    (B)  LOCATION:  Within hemagglutinin

(xi)    SEQUENCE DESCRIPTION:  SEQ ID NO:  8 :

Gly Pro Gly Ala Asn

1                5


INFORMATION FOR SEQ ID NO:  9 :

(i)     SEQUENCE CHARACTERISTICS:

    (A)  LENGTH:  5 amino acids

    (B)  TYPE:  amino acid

    (C)  STRANDEDNESS: _____

    (D)  TOPOLOGY:  unknown

(ii)    MOLECULE TYPE: protein

(v)     FRAGMENT TYPE:  internal

(vi)    ORIGINAL SOURCE: _____

    (A)  ORGANISM:  Influenza Virus

    (B)  STRAIN:  several

(vii)   IMMEDIATE SOURCE: A/Aichi/2/68 influenza virus

(viii)  POSITION IN GENOME: A-antigenic site of hemagglutinin molecule

(ix)   FEATURE: _____

    (A)  NAME/KEY:  <u>SEQ ID NO 9 is part of the</u>

                           <u>influenza virus A-antigenic</u>

                           <u>which is homologous to an HIV</u>

                           <u>principal-neutralizing determinant</u>

    (B)  LOCATION:  <u>Within hemagglutinin</u>

(xi)   SEQUENCE DESCRIPTION:  SEQ ID NO:  <u>9</u> :

Gly Pro Gly Ser

1

(2)  INFORMATION FOR SEQ ID NO:  <u>10</u> :

  (i)     SEQUENCE CHARACTERISTICS:

    (A)  LENGTH:  <u>5 amino acids</u>

    (B)  TYPE:  <u>amino acid</u>

    (C)  STRANDEDNESS:  _____

    (D)  TOPOLOGY:  <u>unknown</u>

  (ii)    MOLECULE TYPE: <u>protein</u>

  (v)     FRAGMENT TYPE:  <u>internal</u>

  (vi)    ORIGINAL SOURCE:  _____

    (A)  ORGANISM:  <u>Influenza Virus</u>

    (B)  STRAIN:  <u>several</u>

  (vii)   IMMEDIATE SOURCE: <u>A/Victoria/3/75 influenza virus</u>

  (viii)  POSITION IN GENOME: <u>A-antigenic site of hemagglutinin molecule</u>

  (ix)    FEATURE:  _____

    (A)  NAME/KEY:  <u>SEQ ID NO 10 is part of the</u>

                           <u>influenza virus A-antigenic site</u>

                           <u>which is homologous to an HIV</u>

                           <u>principal-neutralizing determinant</u>

    (B)  LOCATION:  <u>Within hemagglutinin</u>

(xi) SEQUENCE DESCRIPTION: SEQ ID NO: _10_:

Gly Pro Gly Asp Ser

1    5

(2) INFORMATION FOR SEQ ID NO: _11_:

(i)  SEQUENCE CHARACTERISTICS:

  (A) LENGTH: <u>6 amino acids</u>

  (B) TYPE: <u>amino acid</u>

  (C) STRANDEDNESS: <u>    </u>

  (D) TOPOLOGY: <u>unknown</u>

(ii) MOLECULE TYPE: <u>protein</u>

(v)  FRAGMENT TYPE: <u>internal</u>

(vi) ORIGINAL SOURCE: <u>    </u>

  (A) ORGANISM: <u>Influenza Virus</u>

  (B) STRAIN: <u>several</u>

(vii) IMMEDIATE SOURCE: <u>A/Aichi/2/68 X31 influenza virus</u>

(viii) POSITION IN GENOME: <u>A-antigenic site of hemagglutinin molecule</u>

(ix) FEATURE: <u>    </u>

  (A) NAME/KEY: <u>SEQ ID NO 11 is part of the</u>

         <u>influenza virus A-antigenic site</u>

         <u>which is homologous to an HIV</u>

         <u>principal-neutralizing determinant</u>

  (B) LOCATION: <u>Within hemagglutinin</u>

(xi) SEQUENCE DESCRIPTION: SEQ ID NO: _11_:

Gly Pro Gly Ser Gly Phe

1    5

(2)   INFORMATION FOR SEQ ID NO: _12_ :

    (i)   SEQUENCE CHARACTERISTICS:

        (A)   LENGTH: 12 nucleotides

        (B)   TYPE: nucleic acid

        (C)   STRANDEDNESS: Single

        (D)   TOPOLOGY: Linear

    (ii)   MOLECULE TYPE: mRNA, or DNA

    (v)   FRAGMENT TYPE: internal

    (vi)   ORIGINAL SOURCE:

        (A)   ORGANISM: Human Immunodeficiency Virus

        (B)   STRAIN: several

    (vii)   IMMEDIATE SOURCE: Mutagenesis

    (viii)   POSITION IN GENOME: Within ENV

    (ix)   FEATURE:

        (A)   NAME/KEY: SEQ ID NO 12 is part of the coding sequence for a principal-neutralizing determinant of HIV

        (B)   LOCATION: Within ENV

    (xi)   SEQUENCE DESCRIPTION: SEQ ID NO: _12_ :

GGN CCN GGN CGN                    12

Gly Pro Gly Arg

1

(2)   INFORMATION FOR SEQ ID NO: _13_ :

    (i)   SEQUENCE CHARACTERISTICS:

        (A)   LENGTH: 12 nucleotides

        (B)   TYPE: nucleic acid

        (C)   STRANDEDNESS: Single

        (D)   TOPOLOGY: Linear

    (ii)   MOLECULE TYPE: mRNA, or DNA

    (v)   FRAGMENT TYPE: internal

    (vi)   ORIGINAL SOURCE:

        (A)   ORGANISM: Human Immunodeficiency Virus

        (B)   STRAIN: several

(vii)   IMMEDIATE SOURCE: Mutagenesis

(viii)  POSITION IN GENOME: Within ENV

(ix)    FEATURE: _____

      (A)  NAME/KEY:  SEQ ID NO 13 is part of the

                        coding sequence for a

                        principal-neutralizing

                        determinant of HIV

      (B)  LOCATION:  Within ENV

(xi)    SEQUENCE DESCRIPTION:  SEQ ID NO:  13 :

      GGN CCN GGN AGR                  12

      Gly Pro Gly Arg

      1


(2)   INFORMATION FOR SEQ ID NO:  14 :

    (i)     SEQUENCE CHARACTERISTICS:

      (A)  LENGTH:  12 nucleotides

      (B)  TYPE:  nucleic acid

      (C)  STRANDEDNESS:  Single

      (D)  TOPOLOGY:  Linear

    (ii)    MOLECULE TYPE: mRNA, or DNA

    (v)     FRAGMENT TYPE:  internal

    (vi)    ORIGINAL SOURCE: _____

      (A)  ORGANISM:  Influenza virus

      (B)  STRAIN:  A/Duck/Ukraine/63

    (vii)   IMMEDIATE SOURCE: Viral clone

    (viii)  POSITION IN GENOME: Within HEMAGGLUTININ

    (ix)    FEATURE: _____

      (A)  NAME/KEY:  SEQ ID NO 14 is part of the

                        coding sequence for the

                        A-antigenic site of the

                        influenze hemagglutinin

      (B)  LOCATION:  Within hemagglutinin

    (xi)    SEQUENCE DESCRIPTION:  SEQ ID NO:  14 :

      GGA CCT GCT AAC                  12

      Gly Pro Ala Asn

      1

(2) INFORMATION FOR SEQ ID NO: _15_:

    (i)      SEQUENCE CHARACTERISTICS:

          (A)  LENGTH: _12 nucleotides_____

          (B)  TYPE: _nucleic acid____

          (C)  STRANDEDNESS: _Single_____

          (D)  TOPOLOGY: _Linear_____

    (ii)     MOLECULE TYPE: _mRNA, or DNA_

    (v)      FRAGMENT TYPE: _internal_____

    (vi)     ORIGINAL SORUCE: _____

          (A)  ORGANISM: _Human Immunodeficiency Virus_

          (B)  STRAIN: _several_____

    (vii)    IMMEDIATE SOURCE: _Mutagenesis_____

    (viii)  POSITION IN GENOME: _Within ENV_

    (ix)     FEATURE: _____

          (A)  NAME/KEY: _SEQ ID NO 15 is a nucleotide sequence coding_

                     _for a principal-neutralizing determinant of_

                     _HIV which can be introduced into the influenza_

                     _hemagglutinin through mutagenesis___

          (B)  LOCATION: _Within ENV_____

    (xi)     SEQUENCE DESCRIPTION:  SEQ ID NO: _15_:

          GGA CCT GGT AGR                12

          Gly Pro Gly Arg

          1

(2) INFORMATION FOR SEQ ID NO: _16_:

    (i)      SEQUENCE CHARACTERISTICS:

          (A)  LENGTH: _12 nucleotides_____

          (B)  TYPE: _nucleic acid____

          (C)  STRANDEDNESS: _Single_____

          (D)  TOPOLOGY: _Linear_____

    (ii)     MOLECULE TYPE: _mRNA, or DNA_

(v)      FRAGMENT TYPE:  internal

(vi)     ORIGINAL SOURCE:  _____

      (A)  ORGANISM:  Influenza virus

      (B)  STRAIN:  A/Aichi/2/68

(vii)    IMMEDIATE SOURCE: Viral clone

(viii)   POSITION IN GENOME: Within HEMAGGLUTININ

(ix)     FEATURE:  _____

      (A)  NAME/KEY:  SEQ ID NO 16 is part of the

                       coding sequence for the

                       A-antigenic site of the

                       influenza hemagglutinin

      (B)  LOCATION:  Within hemagglutinin

(xi)     SEQUENCE DESCRIPTION:  SEQ ID NO:  16 :

GGA CCT GGT AGC                    12

Gly Pro Gly Ser

1

(2)  INFORMATION FOR SEQ ID NO:  17 :

    (i)      SEQUENCE CHARACTERISTICS:

        (A)  LENGTH:  12 nucleotides

        (B)  TYPE:  nucleic acid

        (C)  STRANDEDNESS:  Single

        (D)  TOPOLOGY:  Linear

    (ii)     MOLECULE TYPE:  mRNA, or DNA

    (v)      FRAGMENT TYPE:  internal

    (vi)     ORIGINAL SOURCE:  _____

        (A)  ORGANISM:  Human Immunodeficiency Virus

        (B)  STRAIN:  several

    (vii)    IMMEDIATE SOURCE: Mutagenesis

    (viii)   POSITION IN GENOME: Within ENV

    (ix)     FEATURE:  _____

        (A)  NAME/KEY:  SEQ ID NO 17 is part of the

                       coding sequence for a

                       principal-neutralizing

                       determinant of HIV

        (B)  LOCATION:  Within ENV

(xi)   SEQUENCE DESCRIPTION:  SEQ ID NO:  17 :

GGA CCT GGT AGR                    12

Gly Pro Gly Arg

1

(2)   INFORMATION FOR SEQ ID NO:  18 :

    (i)   SEQUENCE CHARACTERISTICS:

       (A)  LENGTH:  12 nucleotides

       (B)  TYPE:  nucleic acid

       (C)  STRANDEDNESS:  Single

       (D)  TOPOLOGY:  Linear

    (ii)   MOLECULE TYPE: mRNA, or DNA

    (v)   FRAGMENT TYPE:  internal

    (vi)   ORIGINAL SOURCE:  _____

       (A)  ORGANISM:  Influenza virus

       (B)  STRAIN:  A/Victoria/3/75

    (vii)   IMMEDIATE SOURCE: Viral clone

    (viii)  POSITION IN GENOME: Within HEMAGGLUTININ

    (ix)   FEATURE:  _____

       (A)  NAME/KEY:  SEQ ID NO 18 is part of the

                 coding sequence for the

                 A-antigenic site of the

                 influenza hemagglutinin

       (B)  LOCATION:  Within hemagglutinin

    (xi)   SEQUENCE DESCRIPTION:  SEQ ID NO:  18 :

GGA CCT GAT AGC                    12

Gly Pro Asp Ser

1

(2)   INFORMATION FOR SEQ ID NO:  19 :

    (i)   SEQUENCE CHARACTERISTICS:

       (A)  LENGTH:  12 nucleotides

       (B)  TYPE:  nucleic acid

       (C)  STRANDEDNESS:  Single

       (D)  TOPOLOGY:  Linear

(ii)   MOLECULE TYPE: mRNA, or DNA

(v)    FRAGMENT TYPE: internal

(vi)   ORIGINAL SOURCE: _____

      (A)  ORGANISM: Human Immunodeficiency Virus

      (B)  STRAIN: several

(vii)  IMMEDIATE SOURCE: Mutagenesis

(viii) POSITION IN GENOME: Within ENV

(ix)   FEATURE: _____

      (A)  NAME/KEY: SEQ ID NO 19 is part of the coding sequence for a principal-neutralizing determinant of HIV

      (B)  LOCATION: Within ENV

(xi)   SEQUENCE DESCRIPTION: SEQ ID NO: 19 :

GGA CCT GGT AGR           12

Gly Pro Gly Arg

1


(2)  INFORMATION FOR SEQ ID NO: 20 :

    (i)    SEQUENCE CHARACTERISTICS:

       (A)  LENGTH: 18 nucleotides

       (B)  TYPE: nucleic acid

       (C)  STRANDEDNESS: Single

       (D)  TOPOLOGY: Linear

    (ii)   MOLECULE TYPE: mRNA, or DNA

    (v)    FRAGMENT TYPE: internal

    (vi)   ORIGINAL SOURCE: _____

       (A)  ORGANISM: Influenza virus

       (B)  STRAIN: A/Aichi/2/68

    (vii)  IMMEDIATE SOURCE: Cloning

    (viii) POSITION IN GENOME: Within Hemagglutinin

    (ix)   FEATURE: _____

       (A)  NAME/KEY: SEQ ID NO 20 is part of the coding sequence for influenza A-antigenic site and is useful as a hybridization probe

       (B)  LOCATION: Within hemagglutinin

```
(xi)   SEQUENCE DESCRIPTION: SEQ ID NO: _20_:

       GGA CCT GGT AGC GGT TTT            18

       Gly Pro Gly Ser Gly Phe

       1           5
```

```
(2)  INFORMATION FOR SEQ ID NO: _21_:

     (i)     SEQUENCE CHARACTERISTICS:

             (A)  LENGTH: 18 nucleotides

             (B)  TYPE: nucleic acid

             (C)  STRANDEDNESS: Single

             (D)  TOPOLOGY: Linear

     (ii)    MOLECULE TYPE: mRNA, or DNA

     (v)     FRAGMENT TYPE: internal

     (vi)    ORIGINAL SOURCE: _____

             (A)  ORGANISM: Human Immunodeficiency Virus

             (B)  STRAIN: several

     (vii)   IMMEDIATE SOURCE: Mutagenesis

     (viii)  POSITION IN GENOME: Within ENV

     (ix)    FEATURE: _____

             (A)  NAME/KEY: SEQ ID NO 21 is a chimaeric, mutagenic olignucleo-
                            tide coding partially for a principal-neutralizing
                            determinant of HIV and partially for the
                            hemagglutinin A-antigenic site of influenze virus:

             (B)  LOCATION: Within ENV of HIV and hemagglutinin
                            of influenza

     (xi)    SEQUENCE DESCRIPTION: SEQ ID NO: _21_:

             GGA CCT GGA AGA GCT TTT            18

             Gly Pro Gly Arg Ala Phe

             1           5
```

## Claims

1. An attenuated or killed influenza virus, and subunit proteins thereof, comprising a Human Immunodeficiency Virus (HIV) Principal Neutralizing Determinant (PND).

2. The virus and proteins of Claim 1 wherein the HIV PND comprises a portion of said virus' hemagglutinin.

3. The virus and hemagglutinin of Claim 2 wherein the HIV PND comprises a portion of said virus' hemagglutinin A-antigenic site.

4. The virus and hemagglutinin of Claim 3 wherein the influenza virus is of the H3 subtype having a hemagglutinin A-antigenic site comprising a sequence selected from -Gly Pro Gly Arg-, -Gly Pro Gly Arg

21

Ala-, -Gly-Gly Pro Gly Arg Ala Phe-, -Gly Pro Gly Arg Val Ile-, and -Gly Pro Gly Arg Ala Ile-.

5. The virus of Claim 3 wherein the selected sequence is preceeded by -Thr Ile-, -Ser Ile-, -Arg Ile-, -His Ile-, -Asp Arg-, -Gln Arg-, or -Thr Lys-.

6. An immunogenic composition comprising a pharmaceutically acceptable carrier, the virus or purified protein of Claim 1, and optionally, additional antiviral, antibiotic, immunomodulatory, or adjuvant compounds thought to be beneficial in the prevention or treatment of influenza or HIV infection or disease.

7. The use of the composition of Claim 6 for the manufacture of a medicament for treating or preventing influenza or HIV infection or disease.

8. A nucleic acid coding for a chimaeric hemagglutinin-HIV PND protein.

9. A chimaeric influenza hemagglutinin protein comprising an HIV-PND, said protein being derived from mutagenized virus or through recombinant expression of a gene encoding said chimaeric protein.

**European Patent Office**

# EUROPEAN SEARCH REPORT

Application Number

EP 91 31 1660
PAGE 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | WO-A-9 103 552 (THE MOUNT SINAI SCHOOL OF MEDICINE OF THE CITY UNIVERSITY OF NEW YORK) * Page 24 lines 17 - 27, page 31 line 29 - page 33 line 20, page 43 line 15 - page 46 line 20, Figure 10 * | 1-3,6-9 | C12N15/44 A61K39/145 A61K39/21 C07K15/00 C12N15/62 |
| Y | BIOLOGICAL ABSTRACTS PHILADELPHIA, PA US ABSTRACT NO 91072438 V.A. PETRENKO ET AL.: 'Directed reconstruction of the influenza virus hemagglutinin gene' & MOLEKULYARNAYA BIOLOGIYA 1990, vol. 24, no 5, pages 1230 -1240 * Abstract * | 1-9 | |
| D,Y | SCIENCE vol. 249, no. 4971, 24 August 1990, WASHINGTON DC, US pages 932 - 935; G.J. LAROSA ET AL.: 'Conserved sequence and structural elements in the HIV-1 principal neutralizing determinant' * Whole article * | 1-9 | |
| D,A | NATURE vol. 289, no. 5796, 29 January 1981, LONDON GB pages 373 - 378; D.C. WILEY ET AL.: 'Structural identification of the antibody-binding sites of Hong Kong influenza haemagglutinin and their involvement in antigenic variation' * whole article * | 1-3,6-9 | TECHNICAL FIELDS SEARCHED (Int. Cl.5) C12N A61K C07K |
| D,Y | PROCEEDINGS OF THE NATIONAL ACADEMY OF SCIENCES OF USA. vol. 86, no. 17, September 1989, WASHINGTON US pages 6768 - 6772; K. JAVAHERIAN ET AL.: 'Principal neutralizing domain of the human immunodeficiency virus type 1 envelope protein' * Whole article * | 1-9 | |

The present search report has been drawn up for all claims

| Place of search | Date of completion of the search | Examiner |
|---|---|---|
| BERLIN | 07 APRIL 1992 | JULIA P. |

EPO FORM 1503 03.82 (P0401)

European Patent Office

# EUROPEAN SEARCH REPORT

Application Number

EP   91 31 1660
PAGE2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| | --- | | |
| D,Y | VACCINE. vol. 8, no. 4, August 1990, GUILDFORD GB pages 308 - 314; S. MULLER ET AL.: 'Antigenic properties and protective capacity of a cyclic peptide corresponding to site A of influenza virus haemagglutinin' * Whole article * | 1-9 | |
| | --- | | |
| A | EP-A-0 311 219 (STICHTING CENTRAAL DIERGENEESKUNDIG INSTITUUT ET AL.) * Whole document * | 4-5 | |
| | ----- | | |
| | | | TECHNICAL FIELDS SEARCHED (Int. Cl.5) |

The present search report has been drawn up for all claims

| Place of search | Date of completion of the search | Examiner |
|---|---|---|
| BERLIN | 07 APRIL 1992 | JULIA P. |

24